# EUROPEAN PATENT SPECIFICATION

(11) **EP 3 645 703 B1**
(45) Date of publication and mention of the grant of the patent: **26.11.2025**
(21) Application number: 18730462.1
(22) Date of filing: 25.05.2018
(51) Int. Cl.: C12N 5/07

(54) **DETECTION AND ISOLATION OF CANCER CELLS FROM BODY FLUIDS BASED ON A GLYCOSYLATION PATTERN AND METHODS OF USE THEREOF**
NACHWEIS UND ISOLIERUNG VON KREBSZELLEN AUS KÖRPERFLÜSSIGKEITEN AUF DER BASIS EINES GLYCOSYLIERUNGSMUSTERS UND VERWENDUNGSVERFAHREN DAFÜR
DÉTECTION ET ISOLEMENT DE CELLULES CANCÉREUSES À PARTIR DE FLUIDES CORPORELS SUR LA BASE D'UN MOTIF DE GLYCOSYLATION ET LEURS MÉTHODES D'UTILISATION

(30) Priority: 25.05.2017 PT 2017110101; 21.05.2018 PT 2018110748
(43) Date of publication of application: 06.05.2020
(73) Proprietor: Instituto de Patologia e Imunologia da Universidade do Porto (IPATIMUP), 4200-135 Porto (PT); IPO-Porto (Instituto Portugues De Oncologia Do Porto, FG, EPE), 4200-072 Porto (PT); INL (Laboratório Ibérico Internacional De Nanotecnologia), 4715-330 Braga (PT)
(72) Inventor: ALEXANDRE FERREIRA, José, 4500-162 Espinho (PT); OLIVEIRA, Marta, 4715-330 Braga (PT); DIEGUEZ, Lorena, 4715-330 Braga (PT); NEVES, Manuel, 4465-280 São Mamede de Infesta (PT); LARA SANTOS, Lúcio, 4200-072 Porto (PT); RIBEIRO, Ricardo, 4420-061 Gondomar (PT); REIS, Celso, 4430-183 Vila Nova De Gaia (PT)
(74) Representative: Ferreira Pinto, Francisca
(86) International application number: PCT/PT2018/050019
(87) International publication number: WO 2018/217116

(56) References cited:
- WO-A1-2017/083582
- WO-A2-2016/014839
- LORENA DIÉGUEZ: "Microfluidic systems for CTC isolation", 27 January 2018 (2018-01-27), XP055496094, Retrieved from the Internet <URL:http://simposiobiopsialiquida.com/wp-content/uploads/2018/02/Dra.LorenaDieguez.pdf> [retrieved on 20180730]
- HADI ESMAEILSABZALI ET AL: "Detection and isolation of circulating tumor cells: Principles and methods", BIOTECHNOLOGY ADVANCES., vol. 31, no. 7, 1 November 2013 (2013-11-01), GB, pages 1063 - 1084, XP055424325, ISSN: 0734-9750, DOI: 10.1016/j.biotechadv.2013.08.016
- MARIJA BALIC ET AL: "Progress in circulating tumor cell capture and analysis: implications for cancer management", EXPERT REVIEW OF MOLECULAR DIAGNOSTICS, vol. 12, no. 3, 1 April 2012 (2012-04-01), GB, pages 303 - 312, XP055378583, ISSN: 1473-7159, DOI: 10.1586/erm.12.12
- SEUNGHYUN JEON ET AL: "High-Purity Isolation and Recovery of Circulating Tumor Cells using Conducting Polymer-deposited Microfluidic Device", THERANOSTICS, vol. 4, no. 11, 1 January 2014 (2014-01-01), AU, pages 1123 - 1132, XP055340332, ISSN: 1838-7640, DOI: 10.7150/thno.9627
- HYEUN JOONG YOON ET AL: "Emerging Role of Nanomaterials in Circulating Tumor Cell Isolation and Analysis", ACS NANO, vol. 8, no. 3, 25 March 2014 (2014-03-25), US, pages 1995 - 2017, XP055368555, ISSN: 1936-0851, DOI: 10.1021/nn5004277
- JILLIAN M. PRENDERGAST ET AL: "Novel anti-Sialyl-Tn monoclonal antibodies and antibody-drug conjugates demonstrate tumor specificity and anti-tumor activity", MABS, 22 February 2017 (2017-02-22), US, pages 1 - 13, XP055365093, ISSN: 1942-0862, DOI: 10.1080/19420862.2017.1290752
- LIMA LUÍS ET AL: "Sialyl-Tn identifies muscle-invasive bladder cancer basal and luminal subtypes facing decreased survival, being expressed by circulating tumor cells and metastases", UROLOGIC ONCOLOGY: SEMINARS AND ORIGINAL INVESTIGATIONS, vol. 35, no. 12, 2017, XP085262147, ISSN: 1078-1439, DOI: 10.1016/J.UROLONC.2017.08.012

## Description

### Technical field of the invention

The present invention refers to an in vitro method for capturing and recovering the circulating tumor cells (CTCs) from a biological sample as defined in the appended claims by promoting of a contact between the biological sample with a functionalized surface, article or device which is coated with a composition comprising an antibody with affinity for the sialyl-Tn antigen (STn). The present invention is based on the use of a composition comprising sialyl-Tn antigen (STn) antibodies, or other agents with affinity for STn, to capture Circulating Tumour Cells (CTCs) in a functionalized surface or article, such as a CTC isolation device, and the biochemical methods to produce this surface and to detach the cancer cells in a viable state using a specific enzymatic treatment.

The composition, surface and methods may be advantageously applied to coating devices to capture CTCs in blood or other body fluids, to improve their efficiency and specificity in isolating CTCs and to improve their accuracy in assessing the risk of metastization, prognostication, monitoring of disease progression, detection of residual disease and evaluation of response to treatment.

Thus, the present invention falls within the technical field of medicine, pharmaceutics and biochemistry.

### State of the art

Circulating tumour cells (CTCs) are cells released from the primary tumour into the blood that harbour genetic and molecular features found in metastatic sites [1].

CTC counts were proven to better predict overall survival than other cancer biomarkers in several models [2].

CTCs are rare, accounting for less than 0.004% of all mononucleated blood cells [3] which makes their isolation a technical challenge [4] .

The development of the first silicon-based microfluidics capture device constituted a break-through in CTC research, presenting an opportunity to overcome the limitations presented by the available methods [3].

These so called Microchips allow not only the separation of CTCs from other blood components based on size, without the need of prior processing, but also the functionalization with antibodies and other ligands, thereby improving binding specificity and CTC purity [5-9].

The majority of existing microfluidics devices selectively isolate cells expressing EpCAM [6,10], a cell-surface protein whose expression is often lost or reduced in metastatic cells [10], resulting in low recovery and sensitivity rates.

Furthermore the pool of isolated cells is often contaminated with blood mononuclear cells and a subtraction of CD45 expressing cells is required.

Approaches exploring other cell-surface markers (such as Vimentin, E-cadherin, CD44) have been attempted but significant contamination with blood cells is still common in these devices, due to the expression of some of these markers by the blood cells, thus hampering their potential as a tool for molecular and clinical assessments.

It is known that the cancer cells found in the tumor tissues have specific alterations in glycosylation and that one of these cancer glycoepitopes includes the sialyl-Tn antigen [12-14].

The term sialyl-Tn (STn) refers to a post-translational modification in cell surface glycoproteins comprising the O-substitution of serine or threonine residues of a given protein with the disaccharide Neu5Ac α-2-6GalNAc.

WO2016/014839 discloses a method for in vitro immunodetection of TAG-72-expressing cancer cells comprising a step of contacting the cancer cells with antibodies binding STn.

WO2017/083582 discloses methods of screening a cell or sample for the presence of at least one STn, wherein said sample is a biological sample obtained from a subject.

The expression of this biomarker in circulating cells (including expression in blood cells, as well as in the tumour cells) and thus, it's potential as bait for the targeted capture of CTCs is totally unexplored in prior art.

Overall, and despite the above mentioned developments, microchips embodying a more metastasis-specific biomarker are still lacking and it is estimated that more aggressive and most relevant CTC population remains under-represented by the use of current detection methods.

It has also become apparent that none of the biomarkers known in the prior art demonstrates a pan-carcinoma profile, with high recovery rates across multiple types of cancer, and performance of existing biomarkers varies significantly amongst cancer types.

Furthermore, the current methods do not allow the retrieval of viable populations of these cells for downstream molecular studies or do so with low efficiency.

These facts are disclosed in order to illustrate the technical problem addressed by the present disclosure.

### Summary of the Invention

The invention is set out in the appended set of claims. It is based in sialyl-Tn antigen (STn) being expressed in virtually all circulating tumour cells (CTCs) isolated from the blood of cancer patients with bladder, head-and-neck and colorectal cancer; to the lack of expression of this antigen in the peripheral blood mononuclear cells (PBMC; CD45+), including T-lymphocytes (CD3+), B-lymphocytes (CD19+) and monocytes (CD14+), and thus to an invention in which STn antibodies are used in a composition for coating CTCs isolation devices, to provide a solution to improve their efficiency and accuracy of isolating and counting CTCs for clinical and research purposes.

The present invention discloses a composition for coating Circulating Tumour Cells (CTCs) isolation devices that comprises a capturing agent for the sialyl-Tn glycan antigen (STn), such as an antibody, or an antibody fragment, or other organic or inorganic molecule with affinity for STn.

The present invention further refers to an article or surface comprising a STn capturing agent such as an antibody, or an antibody fragment, or a lectin, or an aptamer, or other organic or inorganic molecule with affinity for STn.

Another aspect of this invention further refers to the methods to bind such STn capturing agent composition to the article or surface, and a method to detach the cells bound to this surface, which comprises an enzymatic step employing glycoside hydrolase enzymes such as α-neuraminidase.

The use, composition, surface and methods may be advantageously applied to coating devices to capture CTCs from in vitro samples of blood or other body fluids, to improve their efficiency and specificity in isolating CTCs and to improve their accuracy in assessing the risk of metastization, prognostication, monitoring of disease progression, detection of residual disease and evaluation of response to treatment.

Targeting cancer cells through STn allows access to cancer cell subpopulations that do not express epithelial markers, and overcomes limitations related to low number of isolated CTCs and contamination with blood cells, thus providing a more efficient and specific solution to accurate CTC-based disease management.

### Description of the invention

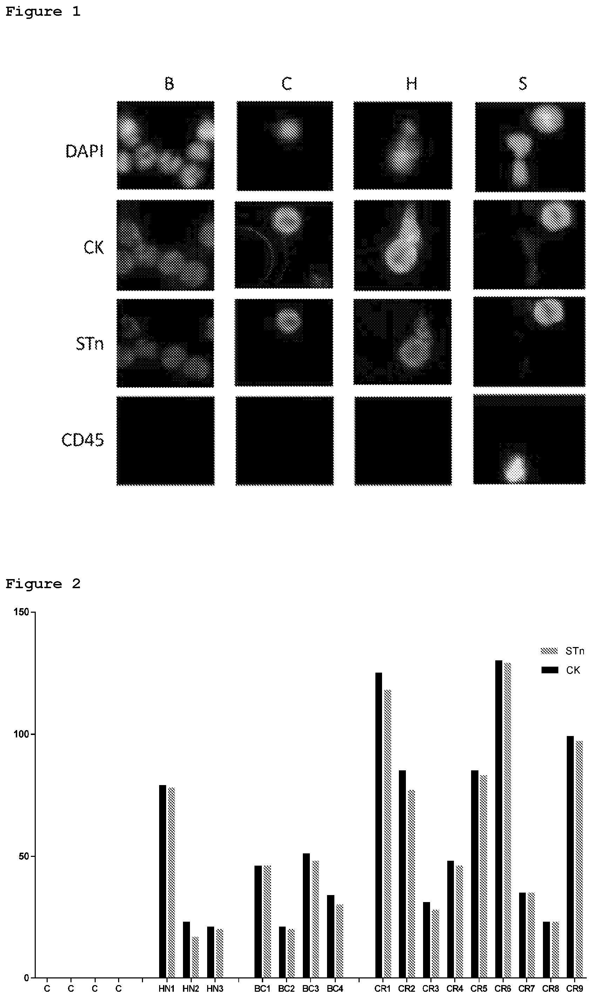

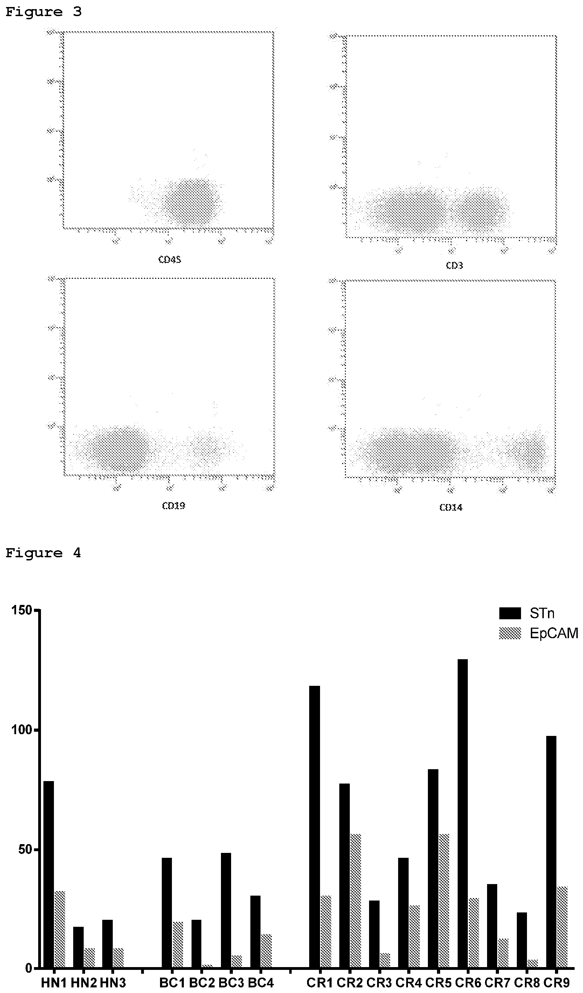

The present invention as defined in the appended claims is based in the use of antibodies against the sialyl-Tn antigen (STn), or other agents with affinity for STn, in a composition for coating CTCs isolation devices, to act as decoys to efficiently capture virtually all blood Circulating Tumour Cells(CTCs) from several types of solid tumours including, but not limited to, bladder, head-and-neck and colorectal cancer, without the undesired binding of peripheral blood mononuclear cells (Figures 1-3) and with increased efficiency relative to epithelial markers (Figure4).

Possible specific antibodies that can be used for the purpose comprise the STn-antibodies B72.3, CC49, STn219, MLS102, TKH2, HB.STn-1,LLU9B4, 3P9, clone B35.1, clone B35.2, clone SWn330, L2A5, all characterized by having affinity for STn, that can be obtained from commercial sources, such as the Abcam, Santa Cruz, Histosearch, Life Span Biosciences, Creative Diagnostics, or other companies.

In other embodiments, fragments of the variable domain of the above mentioned antibodies can be used for the purpose of this invention as defined in the appended set of claims.

In one embodiment of the present invention, the composition for coating a microfluidics CTCs isolation device is prepared by diluting a specific antibody for STn in Phosphate Buffered Saline (PBS) with 2% (w/v) Bovine Serum Albumine (BSA), in amounts ranging from 5-50 micrograms, most preferably 33 micrograms.

CTCs isolation devices for coating with the said compositions can be produced for example from, but not limited to, masters for Polydimethylsiloxane (PDMS) moulding using standard photolithography and soft lithography techniques. Devices can also be fabricated by hot embossing, nano-imprint-lithography, micromachining, laser cutting and other techniques without limitation. In one example of production of a CTC isolation device, cylindrical features with diameters of 100 µm and interspacing of 100 µm are used in a geometric arrangement with a 100 µm shift in every row to maximise mixing. Silicon wafers are cleaned by subsequent sonication steps in acetone and isopropanol for 5 minutes each, dried with nitrogen and activated in oxygen plasma for 5 minutes. Upon activation the wafers are immersed in 5% 3-(Glycidyloxypropyl)trimethoxysilane in ethanol for 1h, rinsed in ethanol and cured at 80°C for 1 hour. SU8-50 is dispensed on the wafer and spun to achieve a 50 µm thick layer. The wafer is soft baked in 2 steps, first at 65°C for 10 minutes and secondly at 95°C for 30 minutes. The wafer is then exposed through a transparency mask to a UV lamp with a dose of 250 mJ/cm2, and postbaked at 55°C overnight. The sample is developed for 20 minutes and hardbaked ramping from 65°C to 200°C. Prior to master replication, the wafer is hydrophobized with a vapour-phase treatment in trichloro 1,1,2,2-perfluorooctyl-silane for 1 hour in a desiccator and cured for another hour at 80°C. PDMS is mixed at 1:10 ratio, degassed, poured over the master, degassed again and cured at 80°C for 2 hours. After curing, the PDMS is unmoulded and the inlet and outlet are punched. Finally, clean glass slides and PDMS replicas are treated with oxygen plasma at low power for 15 seconds and subsequently brought in contact to produce irreversible bonding. Upon activation with oxygen plasma, the microfluidic devices are connected to a syringe pump.

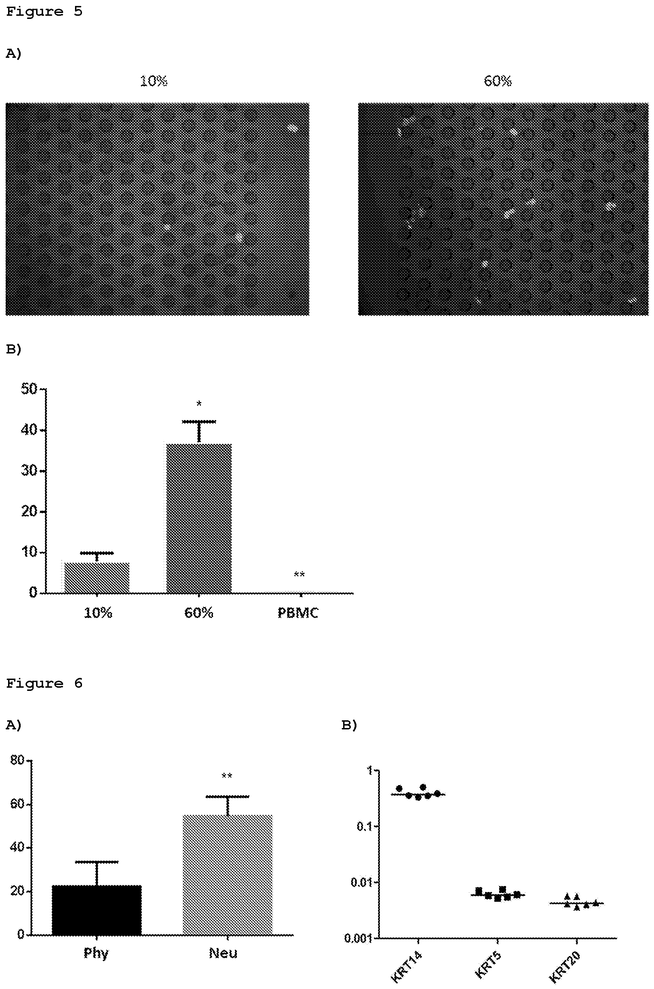

Using the said compositions it is possible to develop an embodiment of the present invention in which a selective functionalized surface, article or device onto which tumour cells in blood may be captured, immobilized or sorted, is produced by binding to the above mentioned compositions, on the basis of the expression of STn (Figure 5).

As such, the present invention further refers to a functionalization method to produce a selective surface, article or device which comprises the steps of:
a) Stabilizing the surface, article or device with ethanol and 2% (3-Aminopropyl)triethoxysilane (APTES);
b) Rinsing with ethanol; followed by deionized water; 1% glutaraldehyde; and again deionized water;
c) Stabilizing with Phosphate Buffered Saline (PBS);
d) Promoting the contact of the said surface, article or device with the said composition for a period of time higher than 1 min, higher than 1 hour, most preferably 8 hours, at a temperature in the range of 4-25°C, most preferably 4°C and;
e) Rinsing the said surface, article or device the with PBS;
f) Blocking the said surface, article or device with 2% bovine serum albumin (BSA) in PBS followed by 1% human serum albumin.

In other embodiments, the said surfaces, articles or devices may comprise silicones, such as polydimethylsiloxane polymer, acrylic or acrylic glasses, such as polymethylmethacrylate acrylic, filters, microfluidics devices, cell sorters, flow cytometers, or any other devices with the purpose of capturing, sorting or isolating cancer cells, without limitation.

Another aspect of the present invention is the ability to recover the CTCs captured onto the said surface, article or device in a viable state, allowing for example *in vitro* culture of these cells, transplantation in in vivo patient-derived xenograft models or the performance of molecular and functional studies (Figure 6).

Thus, in another embodiment of the present invention, a recovery method was developed for detaching the captured CTCs from the said surface, article or device (Figure 6), the said method comprising the steps of:
a) Applying glycoside hydrolase enzymes, such as α-neuraminidase at, for example, a concentration in the range of 0.5 to 5 U/mL, most preferably at 1 U/mL to the said surface, article or device with bound CTCs;
b) Incubating the surface, article or device at 37°C for a period not inferior to 60 minutes;
c) Flowing or washing for recovery of the released cells.

One Unit (U) α-neuraminidase in the previous embodiment is the enzyme activity that hydrolyzes 1 mole of N-acetyl-neuraminosyl-D-lactose in 1 min at +25°C under the following incubation conditions: 10 mM N-acetyl-neuraminosyl-D-lactose, 50 mM sodium acetate, pH 5. The α-neuraminidase can be obtained by methods known in the art from commercial sources such as the company Sigma Aldrich.

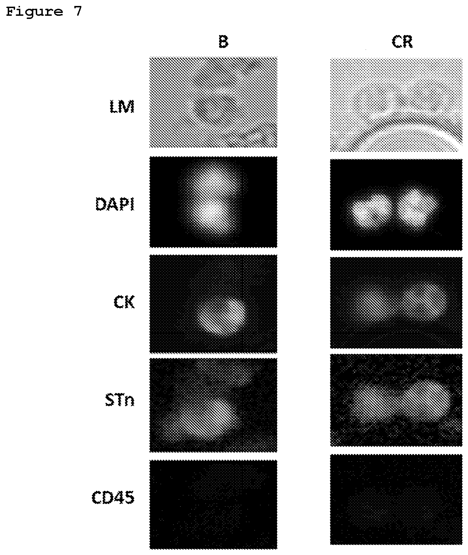

Altogether, another embodiment of the present invention refers to a method for capturing, recovering and assessing the CTCs in a biological sample (Figure 7), the said method comprising the steps of:
a) Preparing the said composition for capture of CTCs;
b) Functionalizing the said surface, article or device, using the said functionalization method;
c) Promotion of a contact between a biological sample obtained in vitro with the said functionalized surface, article or device;
d) Detaching the cells using the said recovery method;
e) Analyzing the recovered sample in terms of the number of cells, or their immunofluorescence expression of STn or other biomarkers, or their in vitro or in vivo properties.

In particular the present invention is directed to an *In vitro* method for capturing and recovering the CTCs from a biological sample characterized by comprising, the steps of:
a) Promotion of a contact between the biological sample with a functionalized surface, article or device;
b) After flow-through of the biological sample, applying glycoside hydrolase enzymes, such as α-neuraminidase at a concentration in the range of 0.5 to 5 U/mL, to the said surface, article or device;
c) Incubating the surface, article or device at 37°C for a period not inferior to 60 minutes;
d) Flowing or washing the surface, article or device for recovery of the released cells, wherein said functionalized surface, article or device is coated with a composition comprising an antibody with affinity for the sialyl-Tn antigen (STn).

In other embodiments, the said biological sample may comprise whole blood, urine, bladder wash, amniotic fluid, peritoneal fluid, pleural fluid, cerebrospinal fluid, lymph, seminal fluid, or a diluted faecal sample.

In conclusion, targeting cancer cells-associated STn allows access to cancer cell subpopulations that do not express the conventionally used epithelial markers, overcomes limitations related with low number of isolated CTCs and with contamination with blood cells and further allows for an efficient recovery process thus providing an overall more efficient and specific solution for accurate CTC assessment.

The said use, composition, surface and methods may be advantageously applied to CTCs isolation devices to significantly improve the efficiency and specificity in both capturing and releasing CTCs and the overall accuracy of assessing CTCs for prognostication, monitoring of disease progression, detection of residual disease and evaluation of response to treatment.

### Brief Description of the Figures

**Figure 1****:** Immunofluorescent expression of sialyl-Tn (STn) in circulating tumour cells isolated from the blood of patients with bladder, head-and-neck and colorectal cancer, according to an example embodiment of the present invention.
   In this figure the first row shows all cell nuclei stained with 4',6-Diamidine-2'-phenylindole dihydrochloride (DAPI). The second row shows circulating tumour cells marked for pan-Citokeratin (CK), a well-established biomarker of CTCs. The third row shows that all the circulating tumour cells are also expressing sialyl-Tn (STn). The forth row shows that circulating tumour cells are negative for hematopoietic marker (CD45). CTCs are defined as DAPI+/CK+/CD45-cells and non-neoplastic blood cells are defined as DAPI+/CK-/CD45+.
   B - Sample from patient with Bladder cancer
   C - Sample from patient with Colorectal cancer
   H - Sample from patient with Head-and-Neck cancer
   S - Sample in which a contamination with a CD45+ cell is observed
**Figure 2****:** Number of sialyl-Tn (STn) positive circulating tumour cells in blood samples from controls, and from bladder, head-and-neck and colorectal cancer patients.
   In this figure it is possible to observe that, despite variation amongst different patients, the CTC count when using either STn is very similar to the CTC count using CK (a well-established marker of CTCs).
   Grey shading: STn CTC count
   Black filling: CK CTC count
   Y axis - Number of CTCs
   X axis: C - Control blood samples from matching age and gender donors without known neoplasia. HN1-3: Blood samples from Head-and-Neck cancer patients 1 to 3.BC1-4: Blood samples from Bladder Cancer patients 1 to 4. CR1-9: Blood samples from ColoRectal cancer patients 1 to 9.
**Figure 3****:** Fluorescence-activated cell sorting (FACS) analysis showing that STn is not expressed by peripheral blood mononuclear cells (PBMC; CD45), including T-lymphocytes (CD3), B-lymphocytes (CD19) and monocytes (CD14) from blood from controls without known neoplasia.
   Y axis - Fluorecence Intensity of STn staining
   X axis - Number of events (cells counted)
**Figure 4****:** CTC counts based in STn and EpCAM staining in blood samples from controls and from bladder, head-and-neck and colorectal cancer patients.
   This figure shows that, despite variance amongst patients, the number of isolated CTCs is higher when using STn relative to the count based on EpCAM, the classical membrane biomarker for CTC isolation and counting.
   Y axis - Number of CTCs
   X axis: HN1-3: Blood samples from Head-and-Neck cancer patients 1 to 3.BC1-4: Blood samples from Bladder Cancer patients 1 to 4. CR1-9: Blood samples from ColoRectal cancer patients 1 to 9.
**Figure 5****:** Binding of cancer cells onto the surface of a functionalized microfluidics device surface according to the levels of STn expression.
   This figure shows that on the surface of microfluidics device functionalized with an anti-STn antibody, the number of cells that are captured from blood samples spiked with cancer cells which have 60% of STn expression, is higher than the number of cells that are captured in blood samples spiked with cancer cells expressing low levels of the antigen (10% of STn expression).
   Panel A shows cancer cells stained with calcein on the functionalized surface of the device.
   Panel B shows a graph quantifying the percentage of STn-positive cells captured from blood spiked with cancer cells with 60% STn expression, cancer cells expressing low levels of sialyl-Tn (10% STn expression) and PBMCs.
   Y axis - Percentage of captured STn cells
   X axis - Samples spiked with cells expressing 10% STn; Samples spiked with cells expressing 60% STn and Samples Peripheral Blood Mononuclear Cells without cancer cells.
**Figure 6****:** Recovery of CTCs from a STn-antibody functionalized surface of a CTC device
   Panel A of this figure shows that recovery the detachment is higher using α-neuraminidase relative to using a conventional physical method based on the recovery of the cells in the inlet by inversion of the flux of cell buffer injection. ** p>0.01 for 3 independent replicates.
   Y axis - Percentage of cell recovery
   X axis - Physical methods (Phy); α-Neuraminidase treatment (Neu) **Panel** B shows that it is feasible to perform studies of mRNA levels of KRT14, KRT5 and KRT20 that are molecular markers used for patient stratification in CTCs recovered from the blood of bladder cancer patients.
   Y axis - Normalized mRNA levels
   X axis - Expression of genes KRT14, KRT5 and KRT20
**Figure 7****:** Isolation of CTCs expressing STn isolated from the blood of bladder and colorectal cancer patients using the microfluidics, according to an example embodiment of the present invention.
   In this figure, the first row shows a Light Microscopy field (LM), second row shows all cell nuclei stained with 4',6-Diamidine-2'-phenylindole dihydrochloride (DAPI). The third row shows circulating tumour cells marked for pan-Citokeratin (CK), a well-established biomarker of CTCs. The forth row shows that all the circulating tumour cells are also expressing sialyl-Tn (STn). The fifth row shows that circulating tumour cells are negative for hematopoietic marker (CD45). CTCs are defined as DAPI+/CK+/STn+/CD45- cells.

### EXAMPLES

### Example 1 - Preparation of the compositions for coating comprising antibodies for STn-carrier protein domains

In one example of the invention, a composition is produced by comprising an antibody targeting domains of a specific protein containing the STn antigen, such as antibodies against STn-containing domains of mucin 1, CD44, integrin β1 or osteopontin.

In this embodiment, the composition for coating a microfluidics CTCs isolation device is prepared by diluting the antibody against STn-containing domains of STn-carrier proteins, exemplified but not limited to mucin 1, CD44, integrin β1 or osteopontin in Phosphate Buffered Saline (PBS) with 2% (w/v) Bovine Serum Albumine (BSA), in amounts ranging from 5-100 micrograms.

The advantage of this embodiment is that targeting the STn antigen in a specific carrier protein may allow specificity for CTCs from a specific type of cancer which overexpress the specific STn-carrier protein.

### Example 2 - Method for coating functionalized surfaces or devices

In one example of the invention, a method for coating to produce a selective surface, article or device comprises the steps of:
a) Stabilizing the surface, article or device with ethanol and 2% (3-Aminopropyl)triethoxysilane (APTES);
b) Rinsing with ethanol; followed by MilliQ water; 1% glutaraldehyde; and again MilliQ water;
c) Stabilizing with Phosphate Buffered Saline (PBS);
d) Promoting the contact of the said surface, article or device with the composition as described in example 2 for a period of time higher than 1 min, higher than 1 hour, most preferably 8 hours, at a temperature in the range of 4-25°C, most preferably 4°C and;
e) Rinsing the said surface, article or device the with PBS;
f) Blocking the said surface, article or device with 2% bovine serum albumin (BSA) in PBS followed by 1% human serum albumin.

### Example 3 - Method for functionalizing surfaces articles or devices comprising other anchoring materials and covalent chemistry

In one example of the invention, a method for functionalization of devices is by attaching an STN-targetting antibody through anchoring a Self-Assembled Monolayer (SAM) containing silane and glutaraldehyde, or by thiols or by other covalent chemistry, or by physisorption, or by polyelectrolytes, among others. Surfaces may comprise silicon-based materials or polymers and may be micro-nano-fluidic systems or other articles/devices, such as, thermoplastic molded devices, made of a polymer having a carboxyl group, made of a polymer having a primary amino group made of Poly(methyl methacrylate), cell sorters, flow cytometers, or any other devices with the purpose of capturing, sorting or isolating cancer cells.

The advantage to use the covalent chemistry in a SAM form, is that the functionalisation is very specific, maximising surface area and minimising non-specific binding.

### Example 4 - Method for capture and recovery of tumor cells from bodily fluids

In one example of the present invention, the isolation of cancer cells may be performed with the above mentioned compositions, selective surfaces of devices and methods on cells shed or actively released into other types of bodily fluids that may comprise urine, bladder wash, peritoneal fluid, pleural fluid, cerebrospinal fluid, seminal fluid, or a diluted faecal sample.

In this example, the advantage is that the compositions, selective devices surface and methods may be used for detecting cancer cells shed in body fluids or diluted biological materials irrespective of their extravasation into the blood circulation, with the potential to encompass also the pre-metastatic stages, allowing detection of cancer cells using non-invasive methods at an earlier stage, with increased chances of patient survival.

### Example 5 - Method for capture and recovery of tumor cell's vesicles

In another example of the present invention, the method may be used to isolate tumour cell's derived extracellular vesicles released from cancer cells.

In this example, the advantage is to provide a pre-enrichment for clinically relevant extracellular vesicles enabling their assessment for diagnosis, prognosis, monitoring of relapse and response to treatment as well as downstream molecular, functional and clinical studies.

### Example 6 - Method for capture and recovery of other STn expressing cells

In another example of the present invention, the methods may be used for isolation of other types of cells expressing the STn, including but not restricted to other mammalian cells, bacteria, parasites and viruses. Examples of fluids of different natures may be bodily fluids, environmental fluids, different culture media, food fluids, and others.

In this example, the advantage is a rapid and easy method for isolation of bacteria and viruses from different fluidsin industries, or for environmental monitoring of pathogens, amongst other applications.

### References:

[1] Onstenk W, Sieuwerts AM, Mostert B, Lalmahomed Z, Bolt-de Vries JB, van Galen A, et al. Molecular characteristics of circulating tumor cells resemble the liver metastasis more closely than the primary tumor in metastatic colorectal cancer. Oncotarget. (2016) 7:59058-59069.
[2]Gorin MA, Verdone JE, van der Toom E, Bivalacqua TJ, Allaf ME, Pienta KJ. Circulating tumour cells as biomarkers of prostate, bladder, and kidney cancer. Nat Rev Urol. (2016) (ahead of print)
[3] Nagrath S, Sequist LV, Maheswaran S, Bell DW, Irimia D, Ulkus L, Smith MR, Kwak EL, Digumarthy S, Muzikansky A, Ryan P, Balis UJ, Tompkins RG, Haber DA, Toner M. Isolation of rare circulating tumour cells in cancer patients by microchip technology. Nature. (2007b) 450:1235-9.
[4] Hong B, Zu Y. Detecting circulating tumor cells: current challenges and new trends. Theranostics. (2013) 3:377-94.
[5] Esmaeilsabzali H, Beischlag TV, Cox ME, Parameswaran AM, Park EJ. Detection and isolation of circulating tumor cells: principles and methods. Biotechnol Adv. (2013) 31:1063-84.
[6] Yoon HJ, Kozminsky M, Nagrath S. Emerging role of nanomaterials in circulating tumor cell isolation and analysis. ACS Nano. (2014) 8:1995-2017.
[7] Sarioglu AF, Aceto N, Kojic N, Donaldson MC, Zeinali M, Hamza B, Engstrom A, Zhu H, Sundaresan TK, Miyamoto DT, Luo X, Bardia A, Wittner BS, Ramaswamy S, Shioda T, Ting DT, Stott SL, Kapur R, Maheswaran S, Haber DA, Toner M. A microfluidic device for label-free, physical capture of circulating tumor cell clusters. Nat Methods. (2015) 12:685-91.
[8] Warkiani ME, Khoo BL, Wu L, Tay AK, Bhagat AA, Han J, Lim CT. Ultra-fast, label-free isolation of circulating tumor cells from blood using spiral microfluidics. Nat Protoc. (2016) 11(1):134-48.
[9] Murlidhar V, Rivera-Báez L, Nagrath S. Affinity Versus Label-Free Isolation of Circulating Tumor Cells: Who Wins? Small. (2016) 12:4450-63. Nagrath S, Sequist LV, Maheswaran S, Bell DW, Irimia D, Ulkus L, et al. Isolation of rare circulating tumour cells in cancer patients by microchip technology. Nature. (2007a) 450:1235-9.
[10] Jeon S, Hong W, Lee ES, Cho Y. High-purity isolation and recovery of circulating tumor cells using conducting polymer-deposited microfluidic device. Theranostics. (2014) 4:1123-32.
[11] Gorges TM, Tinhofer I, Drosch M, Röse L, Zollner TM, Krahn T, von Ahsen O. Circulating tumour cells escape from EpCAM-based detection due to epithelial-to-mesenchymal transition. BMC Cancer. (2012) 12:178.
[12] Marcos NT, Pinho S, Grandela C, Cruz A, Samyn-Petit B, Harduin-Lepers A, Almeida R, Silva F, Morais V, Costa J, Kihlberg J, Clausen H, Reis CA. Role of the human ST6GalNAc-I and ST6GalNAc-II in the synthesis of the cancer-associated sialyl-Tn antigen. Cancer Res. 2004 Oct 1;64(19):7050-7.
[13] Marcos NT, Bennett EP, Gomes J, Magalhaes A, Gomes C, David L, Dar I, Jeanneau C, DeFrees S, Krustrup D, Vogel LK, Kure EH, Burchell J, Taylor-Papadimitriou J, Clausen H, Mandel U, Reis CA. ST6GalNAc-I controls expression of sialyl-Tn antigen in gastrointestinal tissues. Front Biosci (Elite Ed). 2011 Jun 1;3:1443-55.
[14] Ferreira JA, Magalhães A, Gomes J, Peixoto A, Gaiteiro C, Fernandes E, Santos LL, Reis CA. Protein glycosylation in gastric and colorectal cancers: Toward cancer detection and targeted therapeutics. Cancer Lett. (2017) 387:32-45.

## Claims

1. In vitro method for capturing and recovering the circulating tumor cells (CTCs) from a biological sample **characterized by** comprising, the steps of:
a) Promotion of a contact between the biological sample with a functionalized surface, article or device;
b) After flow-through of the biological sample, applying glycoside hydrolase enzymes, such as α-neuraminidase at a concentration in the range of 0.5 to 5 U/mL, to the said surface, article or device;
c) Incubating the surface, article or device at 37°C for a period not inferior to 60 minutes;
d) Flowing or washing the surface, article or device for recovery of the released cells,
wherein said functionalized surface, article or device is coated with a composition comprising an antibody with affinity for the sialyl-Tn antigen (STn).

2. Method according to claim 1, wherein the biological sample comprises whole blood, urine, bladder washes, amniotic fluid, peritoneal fluid, pleural fluid, cerebrospinal fluid, lymph, seminal fluid, or a diluted fecal sample.

3. Method according to claims 1 to 2, wherein said functionalized surface, article or device coated with a composition comprising an antibody with affinity for the sialyl-Tn antigen (STn) is obtainable by a method comprising the steps of:
a) Stabilizing the surface, article or device with ethanol and 2% (3-Aminopropyl)triethoxysilane (APTES);
b) Rinsing with ethanol; deionized water; followed by 1% glutaraldehyde; and again deionized water;
c) Stabilizing with Phosphate Buffered Saline (PBS);
d) Promoting the contact of the said surface, article or device with a composition comprising an antibody with affinity for the sialyl-Tn antigen (STn), for a period of time higher than 1 min, at a temperature in the range of 4-25°C;
e) Rinsing the said surface, article or device with Phosphate Buffered Saline (PBS);
f) Blocking the said surface, article or device with 2% bovine serum albumin (BSA) in Phosphate Buffered Saline (PBS) followed by 1% human serum albumin.

4. Method according to claim 3 wherein said functionalized surface, article or device coated with a composition comprising an antibody with affinity for the sialyl-Tn antigen (STn) is made of a material selected from: silicone, such as polydimethylsiloxane polymer; an acrylic, such aspolymethylmethacrylate; a plastic; a glass; a ceramic or combinations thereof.

5. Method according to claims 3 to 4 wherein said functionalized surface, article or device coated with a composition comprising an antibody with affinity for the sialyl-Tn antigen (STn) is a device for isolating Circulating Tumor Cells (CTCs).

6. Method according to any preceding claim, wherein promotion of contact between the biological sample and the functionalized article is made by means of a surface coated with a composition comprising an antibody with affinity for the sialyl-Tn antigen (STn) present in the functionalized surface, article or device.

7. Method according to claims 1 to 3 wherein said functionalized surface, article or device is coated with a composition comprising an antibody with affinity for the sialyl-Tn antigen (STn), selected from the group consisting of: B72.3, CC49, STn219, MLS102, TKH2, HB.STn-1, LLU9B4, 3P9, B35.1, clone B35.2, SW330, L2A5 and a fragment of the variable domain of the said anti-STn antibodies.

8. Method according to claim 7, wherein the composition for coating the functionalized surface, article or device further comprises at least one of the following components: a buffer, a blocking agent, a preservative, a surface activator, a coating or mixtures thereof.

9. An in vitro method for capturing, recovering and assessing the circulating tumor cells (CTCs) in a biological sample, the said method comprising the steps of:
a) Preparing a composition for capture of circulating tumor cells (CTCs) comprising an antibody with affinity for the sialyl-Tn antigen (STn);
b) Functionalizing a surface, article or device comprising the steps of:
i) Stabilizing the surface, article or device with ethanol and 2% (3-Aminopropyl)triethoxysilane (APTES);
ii) Rinsing with ethanol; deionized water; followed by 1% glutaraldehyde; and again deionized water;
iii) Stabilizing with Phosphate Buffered Saline (PBS);
iv) Promoting the contact of the said surface, article or device with the composition comprising an antibody with affinity for the sialyl-Tn antigen (STn), for a period of time higher than 1 min, at a temperature in the range of 4-25°C;
v) Rinsing the said surface, article or device with Phosphate Buffered Saline (PBS);
vi) Blocking the said surface, article or device with 2% bovine serum albumin (BSA) in Phosphate Buffered Saline (PBS) followed by 1% human serum albumin.
c)Promotion of a contact between a biological sample obtained in vitro with the said functionalized surface, article or device;
d) Detaching the cells comprising the steps of:
i) Applying glycoside hydrolase enzymes, such as α-neuraminidase at a concentration of in the range of 0.5 to 5U/mL to the said surface, article or device with bound circulating tumor cells (CTCs);
ii) Incubating the surface, article or device at 37°C for a period not inferior to 60 minutes;
iii)Flowing or washing for recovery of the released cells.
e)Analysing the recovered sample in terms of the number of cells, or their immunofluorescence expression of sialyl-Tn antigen (STn) or other biomarkers, or their in vitro or in vivo properties.

## Patentansprüche

1. *In-vitro* -Verfahren zum Einfangen und Gewinnen zirkulierender Tumorzellen (CTCs) aus einer biologischen Probe, **dadurch gekennzeichnet, dass** es die folgenden Schritte umfasst:
(a) Förderung des Kontakts zwischen der biologischen Probe und einer funktionalisierten Oberfläche, einem funktionalisierten Gegenstand oder einer funktionalisierten Vorrichtung;
(b) Nach dem Fließen der biologischen Probe Aufbringen von Glykosidhydrolase-Enzymen, wie etwa α - Neuraminidase, in einer Konzentration zwischen 0,5 und 5 U/ml auf die Oberfläche, den Artikel oder das Gerät;
(c) Inkubieren Sie die Oberfläche, den Gegenstand oder das Gerät mindestens 60 Minuten lang bei 37 °C;
d) Abspülen oder Waschen der Oberfläche, des Gegenstands oder der Vorrichtung zur Rückgewinnung der freigesetzten Zellen, wobei die funktionalisierte Oberfläche, der Gegenstand oder die Vorrichtung mit einer Zusammensetzung beschichtet ist, die einen Antikörper mit einer Affinität zum siaIy1- Tn- Antigen ( STn ) umfasst.

2. Verfahren nach Anspruch 1, wobei die biologische Probe Vollblut, Urin, Blasenspülungen, Fruchtwasser, Peritonealflüssigkeit, Pleuraflüssigkeit, Zerebrospinalflüssigkeit, Lymphe, Samenflüssigkeit oder eine verdünnte Stuhlprobe umfasst.

3. Verfahren nach den Ansprüchen 1 bis 2, wobei die funktionalisierte Oberfläche, der Artikel oder die Vorrichtung, die mit einer Zusammensetzung beschichtet ist, die einen Antikörper mit einer Affinität für das sia1y1-Tn-Antigen (STn) umfasst, durch ein Verfahren erhältlich ist, das die folgenden Schritte umfasst:
(a) Stabilisierung der Oberfläche, des Gegenstands oder der Vorrichtung mit Ethanol und 2(3-Aminopropyl) triethoxysilan (APTES);
b) Spülen mit Ethanol, deionisiertem Wasser, anschließend Glutaraldehyd und erneut deionisiertem Wasser;
(c) Stabilisierung mit phosphatgepufferter Kochsalzlösung (PBS);
d) Förderung des Kontakts der Oberfläche, des Artikels oder der Vorrichtung mit einer Zusammensetzung, die einen Antikörper mit einer Affinität für das sia1yI- Tn (ST n)-Antigen umfasst, für einen Zeitraum von mehr als 1 Minute bei einer Temperatur zwischen 4 und 25°C;
(e) Spülen Sie die Oberfläche, den Artikel oder das Gerät mit phosphatgepufferter Kochsalzlösung (PBS);
f) Blockieren der Oberfläche, des Artikels oder der Vorrichtung mit 2 % Rinderserumalbumin (BSA) in phosphatgepufferter Kochsalzlösung (PBS), gefolgt von 1 % Humanserumalbumin.

4. Verfahren nach Anspruch 3, wobei die funktionalisierte Oberfläche, der Artikel oder die Vorrichtung, die mit einer Zusammensetzung beschichtet ist, die einen Antikörper mit Affinität zum siaIyI-Tn (STn)-Antigen umfasst, aus einem Material besteht, das ausgewählt ist aus: Silikon, wie etwa einem Polydimethylsiloxanpolymer; einem Acryl, wie etwa Polymethylmethacrylat; einem Kunststoff; einem Glas; einer Keramik oder Kombinationen davon.

5. Verfahren nach den Ansprüchen 3 bis 4, wobei die funktionalisierte Oberfläche, der Artikel oder die Vorrichtung, die mit einer Zusammensetzung beschichtet ist, die einen Antikörper mit einer Affinität zum sia1y1-Tn-Antigen (STn) umfasst, eine Vorrichtung zum Isolieren zirkulierender Tumorzellen (CTCs) ist.

6. Verfahren nach einem der vorhergehenden Ansprüche, wobei die Förderung des Kontakts zwischen der biologischen Probe und dem funktionalisierten Gegenstand mittels einer Oberfläche erfolgt, die mit einer Zusammensetzung beschichtet ist, die einen Antikörper mit einer Affinität für das in der funktionalisierten Oberfläche, dem Gegenstand oder der Vorrichtung vorhandene sialyl-Tn(STn)-Antigen umfasst.

7. Verfahren nach den Ansprüchen 1 bis 3, wobei die funktionalisierte Oberfläche, der Artikel oder das Gerät mit einer Zusammensetzung beschichtet ist, die einen Antikörper mit einer Affinität für das Antigen sialyl- Tn ( STn ) ausgewählt aus der Gruppe bestehend aus: B 72.3, CC49, STn219, MLS 102, TKH2, HB.STn -1, LLU9B4, 3P9, B35.1, Klon B35.2, SW330, L2A5 und ein Fragment der variablen Domäne der Anti- STn- Antikörper umfasst.

8. Verfahren nach Anspruch 7, wobei die Beschichtungszusammensetzung der funktionalisierten Oberfläche, des Artikels oder der Vorrichtung ferner mindestens eine der folgenden Komponenten umfasst: einen Puffer, ein Blockierungsmittel, ein Konservierungsmittel, einen Oberflächenaktivator, eine Beschichtung oder Mischungen davon.

9. Ein *In-vitro -Verfahren* zum Erfassen, Wiedergewinnen und Auswerten zirkulierender Tumorzellen (CTCs) in einer biologischen Probe, wobei das Verfahren die folgenden Schritte umfasst:
a) Herstellung einer Zusammensetzung zum Einfangen zirkulierender Tumorzellen (CTCs), die einen Antikörper mit einer Affinität zum siaIyI - Tn ( STn )-Antigen umfasst;
b) Funktionalisieren einer Oberfläche, eines Artikels oder eines Geräts, umfassend die folgenden Schritte:
i. Stabilisieren Sie die Oberfläche, den Artikel oder das Gerät mit Ethanol und 2(3-Aminopropyl) triethoxysilan (APTES);
ii. Spülung mit Ethanol, deionisiertes Wasser, gefolgt von Glutaraldehyd und erneut deionisiertem Wasser;
iii. Stabilisierung mit phosphatgepufferter Kochsalzlösung (PBS);
iv. Förderung des Kontakts der Oberfläche, des Artikels oder der Vorrichtung mit der Zusammensetzung, die einen Antikörper mit einer Affinität für das sia1y1-Tn(STn)-Antigen umfasst, für einen Zeitraum von mehr als 1 Minute bei einer Temperatur zwischen 4 und 25°C;
v. Spülen Sie die besagte Oberfläche, den Artikel oder das Gerät mit phosphatgepufferter Kochsalzlösung (PBS);
vi. Blockieren der betreffenden Oberfläche, des Artikels oder Geräts mit 2 % Rinderserumalbumin (BSA) in phosphatgepufferter Kochsalzlösung (PBS), gefolgt von 100% menschlichem Serumalbumin;
(c) Förderung des Kontakts zwischen einer *in vitro gewonnenen biologischen Probe* und der funktionalisierten Oberfläche, dem funktionalisierten Gegenstand oder der funktionalisierten Vorrichtung;
d) Ablösen der Zellen, umfassend die folgenden Schritte:
(i) Aufbringen von Glykosidhydrolase-Enzymen wie Neuraminidase in einer Konzentration zwischen 0,5 und 5 U/ ml auf die Oberfläche, den Artikel oder das Gerät, das gebundene zirkulierende Tumorzellen (CTCs) enthält;
(ii) Inkubieren Sie die Oberfläche, den Artikel oder das Gerät mindestens 60 Minuten lang bei 37 °C;
iii) Laufen lassen oder waschen, um freigesetzte Zellen zurückzugewinnen;
e) Analyse der gewonnenen Probe hinsichtlich der Zellzahl oder ihrer Expression durch Immunfluoreszenz des siaIyI-Tn-Antigens (STn) oder anderer Biomarker oder ihrer *in vitro-* oder in vivo-Eigenschaften.

## Revendications

1. Méthode *in vitro* de capture et de récupération de cellules tumorales circulantes (CTC) à partir d'un échantillon biologique, **caractérisé en ce qu'**il comprend les étapes suivantes :
a) Promotion d'un contact entre l'échantillon biologique avec une surface, un article ou un dispositif fonctionnalisé;
b) Après l'écoulement de l'échantillon biologique, application d'enzymes glycoside hydrolases, telles que l'α-neuraminidase à une concentration comprise entre 0,5 et 5 U/mL, sur ladite surface, ledit article ou ledit dispositif;
c) Incuber la surface, l'article ou le dispositif à 37 ° C pendant une durée non inférieure à 60 minutes;
d) Écoulement ou lavage de la surface, de l'article ou du dispositif pour la récupération des cellules libérées, dans lequel ladite surface, ledit article ou ledit dispositif fonctionnalisé est revêtu d'une composition comprenant un anticorps ayant une affinité pour l'antigène siaIy1-Tn (STn).

2. Méthode selon la revendication 1, dans lequel l'échantillon biologique comprend du sang total, de l'urine, des lavages de vessie, du liquide amniotique, du liquide péritonéal, du liquide pleural, du liquide céphalo-rachidien, de la lymphe, du liquide séminal ou un échantillon fécal dilué.

3. Méthode selon les revendications 1 à 2, dans lequel ladite surface, ledit article ou ledit dispositif fonctionnalisé, revêtu d'une composition comprenant un anticorps ayant une affinité pour l'antigène sialyl-Tn (STn), peut être obtenu par un procédé comprenant les étapes suivantes:
a) Stabilisation de la surface, de l'article ou du dispositif avec de l'éthanol et du 2 (3-aminopropyl) triéthoxysilane (APTES);
b) Rinçage à l'éthanol; eau déionisée; suivi de 1 glutaraldéhyde; et à nouveau d'eau déionisée;
c) Stabilisation avec du tampon phosphate salin (PBS);
d) Favori le contact de ladite surface, de l'article ou du dispositif avec une composition comprenant un anticorps ayant une affinité pour l'antigène sia1yI-Tn (ST n) pendant une durée supérieure à 1 min, à une température comprise entre 4 et 25 0 C;
e) Rincer ladite surface, article ou dispositif avec du phosphate Solution saline tamponnée (PBS);
f) Blocage de ladite surface, de l'article ou du dispositif avec 2 % d'albumine sérique bovine (BSA) dans du tampon phosphate salin (PBS) suivi de 1 % d'albumine sérique humaine.

4. Méthode selon la revendication 3, dans lequel ladite surface, ledit article ou ledit dispositif fonctionnalisé revêtu d'une composition comprenant un anticorps ayant une affinité pour l'antigène siaIyI-Tn (STn) est constitué d'un matériau choisi parmi: le silicone, tel qu'un polymère de polydiméthylsiloxane; un acrylique, tel que le polyméthacrylate de méthyle; un plastique; un verre; une céramique ou des combinaisons de ceux-ci.

5. Méthode selon les revendications 3 à 4 dans lequel ladite surface, ledit article ou ledit dispositif fonctionnalisé revêtu d'une composition comprenant un anticorps ayant une affinité pour l'antigène sialyl-Tn (STn) est un dispositif permettant d'isoler des cellules tumorales circulantes (CTC).

6. Méthode selon l'une quelconque des revendications précédentes, dans lequel la promotion du contact entre l'échantillon biologique et l'article fonctionnalisé est réalisée au moyen d'une surface revêtue d'une composition comprenant un anticorps ayant une affinité pour l'antigène sialyl-Tn (STn) présent dans la surface, l'article ou le dispositif fonctionnalisé.

7. Méthode selon les revendications 1 à 3, dans lequel ladite surface, ledit article ou ledit dispositif fonctionnalisé est revêtu d'une composition comprenant un anticorps ayant une affinité pour l'antigène sialyl-Tn (STn) choisi dans le groupe constitué de: B 72.3, CC49, STn219, MLS 102, TKH2, HB.STn-1, LLU9B4, 3P9, B35.1, clone B35.2, SW330, L2A5 et un fragment du domaine variable desdits anticorps anti-STn.

8. Méthode selon la revendication 7, dans lequel la composition de revêtement de la surface, de l'article ou du dispositif fonctionnalisé comprend en outre au moins l'un des composants suivants : un tampon, un agent de blocage, un conservateur, un activateur de surface, un revêtement ou des mélanges de ceux-ci.

9. Méthode *in vitro* de capture, de récupération et d'évaluation des cellules tumorales circulantes (CTC) dans un échantillon biologique, ledit procédé comprenant les étapes suivantes:
a) Préparation d'une composition pour la capture de cellules tumorales circulantes (CTC) comprenant un anticorps ayant une affinité pour l'antigène siaIyI-Tn (STn);
b) Fonctionnaliser une surface, un article ou un dispositif comprenant les étapes suivantes:
i. Stabiliser la surface, l'article ou le dispositif avec de l'éthanol et 2 (3-Aminopropyl)triéthoxysilane (APTES);
ii. Rinçage à l'éthanol; eau déionisée; suivi de 1 glutaraldéhyde; et à nouveau eau déionisée;
iii. Stabilisation avec du tampon phosphate salin (PBS);
iv. Favoriser le contact de ladite surface, de l'article ou du dispositif avec la composition comprenant un anticorps ayant une affinité pour l'antigène sialyl-Tn (ST n) pendant une durée supérieure à 1 min, à une température comprise entre 4 et 25 0 C;
v. Rincer ladite surface, article ou dispositif avec du phosphate Solution saline tamponnée (PBS) ;
vi. Blocage de la surface, de l'article ou du dispositif en question avec 2 % d'albumine sérique bovine (BSA) dans du tampon salin au phosphate (PBS) suivi de 100 % d'albumine sérique humaine.
c) Promotion d'un contact entre un échantillon biologique obtenu *in vitro* avec ladite surface, article ou dispositif fonctionnalisé;
d) Détachement des cellules comprenant les étapes suivantes :
i) Appliquer des enzymes glycoside hydrolase, telles que la neuraminidase à une concentration comprise entre 0,5 et 5 U/mL sur la surface, l'article ou le dispositif contenant des cellules tumorales circulantes (CTC) liées;
ii) Incuber la surface, l'article ou le dispositif à 37 ° C pendant une période non inférieure à 60 minutes ;
iii) Faire couler ou laver pour récupérer les cellules libérées;
e) Analyse de l'échantillon récupéré en termes de nombre de cellules, ou de leur expression par immunofluorescence de l'antigène siaIyI-Tn (STn) ou d'autres biomarqueurs, ou de leurs propriétés *in vitro* ou in vivo.
